# EUROPEAN PATENT APPLICATION

(11) **EP 2 962 705 A1**
(43) Date of publication of application: **06.01.2016**
(21) Application number: 15166224.4
(22) Date of filing: 04.05.2015
(51) Int. Cl.: A61L 29/04, A61M 25/10

(54) **METHOD FOR MANUFACTORING A BALLOON CATHETER**

(30) Priority: 23.06.2014 US 201462015541 P
(71) Applicant: Biotronik AG, 8180 Bülach (CH)
(72) Inventor: Schwitzer, Alwin, 8180 Bülach (CH); Wesselmann, Matthias, 8455 Rüdlingen (CH); Quint, Bodo, 72908 Dettighofen (CH)
(74) Representative: Randoll, Sören

(57) **Abstract**

The invention is related to balloon, in particular balloons suitable for use as a component of a catheter, and a corresponding method for manufacturing such balloons.

The balloon is made of a compound comprising at least one polymer and at least one crosslinking agent, which is activated by irradiation with UV light.

## Description

The invention is related to balloon, in particular balloons suitable for use as a component of a catheter, and a corresponding method for manufacturing such balloons.

Angioplasty is the technique of mechanically widening narrowed or obstructed arteries, the latter typically being a result of atherosclerosis. An empty and collapsed balloon on a guide wire, known as a balloon catheter, is passed into the narrowed locations and then inflated to a fixed size using water pressures some 75 to 500 times normal blood pressure (6 to 20 atmospheres). The balloon forces expansion of the inner white blood cell/clot plaque deposits and the surrounding muscular wall, opening up the blood vessel for improved flow, and the balloon is then deflated and withdrawn. A stent may or may not be inserted at the time of ballooning to ensure the vessel remains open.

A typical field of use of such balloon catheters is an angioplasty procedure of obstructed coronary arteries. Such arteries have a small diameter and are often tortuous. Therefore it is necessary, that the balloon catheters, e.g. the balloon, exhibit a good compliance. This ensures good deliverability due to the flexibility of the material.

On the other hand, a reduction of the compliance at high dilation pressure is required to ensure a full widening of the arteries. Thereby the balloon should withstand high dilation pressure without bursting (defined as burst pressure).

Consequently, the object of the invention is a method for manufacturing a balloon or balloon catheter for medical use with a good compliance features at low pressure (6-14 bar) and a basically non-compliant behavior in a high pressure range (14-20 bar up to 40 bar).

Another object of the invention is balloon made of a material, which exhibits no substantial losses of monomer or dimer particles.

The object of the invention is achieved by a method for manufacturing a balloon catheter, in particular usable for introducing into a body vessel, whereby the balloon is made of a mixture of at least one polymer and the least one crosslinking agent, characterized in that the method according to the invention comprises a step, whereby the balloon is irradiated by UV light.

If one would analyze a balloon made of polymers as known in the art on a microscopic scale, one would recognize an arrangement of several individual, entangled polymer chains. These chains could slide with respect to each other. After a certain elongation of the chains (= elastic response), the chains start to glide in respect to each other and the balloon material behaves as a viscous liquid. This phenomenon for viscoelastic polymers is called creep. If the elongation of the balloon is above a certain limit, the balloon material starts to creep and therefore the balloon wall thickness becomes irreversible smaller. The interactive force between the polymer chains or the glidability of the chains defines the creep /compliant behavior of the balloon.

According to the invention the balloon is made of a compound comprising at least one polymer and at least one crosslinking agent. The crosslinking agent/s, in the final cured, crosslinked state, is/are chemically bound to parts of individual, separate polymer chains. The crosslinking agent of the invention could be activated by irradiation with UV light. The UV light irradiation activates at least a part of the crosslinking agent, which leads to a reactive group or end of the crosslinking agent. This reactive group likely reacts with the polymer chains, another active group of another crosslinking agent. Thereby several polymer chains or layers of polymer chains connect chemically by the crosslinking agents.

The crosslinking agents allow of the elastic behavior of the polymer chains with respect to each other up to a certain level. Above this level the movement is blocked and the creep of the polymer material is stopped. The crosslinking agents restrict the glidability of the layers or chains above a certain level, whereby the compliance of the material is restricted above a certain level. This leads to desired compliance in the low pressure regime and a restricted compliant behavior in the high pressure regime.

By irradiating with UV light as herein suggested the balloon material is less prone to creeping under high pressure. Also, the maximum pressure before bursting is significantly increased resulting in an elevated security in handling. Further, the radial and axial compliance of the material decreased. Also, as a result of crosslinking the material the content of low molecular weight constituents is reduced minimizing leakage from the balloon material into the body of the patient.

The irradiation by UV light is a cheap and simple method to activate suitable crosslinking agents. The balloon material is not destroyed or otherwise affected except the linked polymer layers or chains.

Additionally, the crosslinking agent hinders monomers and dimers to diffuse out of the balloon material. Avoiding the diffusion of monomers and dimers of the polymer base groups is another great advantage of the invention. The polymers used for balloons are usually synthetic. The diffusion of such compounds out of the balloon into the body vessel is undesirable. The incorporation of such synthetic compounds into the patient is avoided by the present invention.

According to a preferred embodiment of the invention the balloon is formed by blow moulding of a balloon preform. The formed balloon is fastened to a shaft of said catheter and irradiated by UV light. It is advantageous to produce the preform by an extrusion process, in particular by extruding granulate material of a compound comprising at least one polymer and at least one crosslinking agent.

According to a preferred embodiment of the invention a chemical compound consisting of at least one polymer and one crosslinking agent is formed. Such a chemical compound could be easily produced in a standard "compounding step" as granulate material. The granulate material could be used as raw material in an extrusion process. Thereby a balloon preform is produced. The preform is basically formed as a tube. In a blow moulding process the balloon is made out of the preform. The formed balloon is finally fastened to the respective shaft or tube of the catheter. The balloon could be fastened to the shaft by welding or by an adhesive bound. After the fastened balloon, including the adhesive bound, is finally UV radiated to crosslink the polymer of the balloon and improve the adhesion force of the adhesive bound.

In a preferred embodiment the balloon is adhesively bonded to a shaft of said catheter, whereby the adhesive bonding is achieved by UV light irradiation of a suitable compound, in particular an adhesive agent activated or hardened by UV light, preferably using a radical mechanism.

The presence of cross linkers within the polymer matrix does also create the opportunity to promote adhesion to radicalic curing systems in contact with the Polymer. Depending on the compatibility of the cross linker with the polymer matrix conditions can be created in which the cross linkers will concentrate on the polymer surface. This effect is given if the cross linker does show limited physical compatibility with the polymer matrix. In case of an acrylate uv adhesive additional reactive structures are already present at the glueing interface and act as chemical active adhesion promoter.

Especial for demanding balloon dilatations such as CTO or below the knee vessle dilations the technical trend do show benefits to modify the balloon surface by a lubricious hydrophilic coating. The immobilization of these coatings is important since poor adhesion is related with the risk to generate particles within the blood stream.

The presence of cross linkers on the polymer surface can improve the adhesion of the coating. Most beneficial is this mechanism if the curing of the hydrophilic coating itself is given by a radicalic polymerization or cross linking. A synergy is given if the application of the hydrophilic coating itself requires UV curing as given for actual hydrophilic coating systems of the companies Surmodics, Harland or DSM.

The at least one polymer is advantageously selected from the group comprising: PET (Polyethylene terephthalate), PEBA (polyether block amide, e.g Vestamid E or PEBAX,), in particular a polyamide, in particular polyamide 12, nylon, polyester copolymers, blends of polyesters or blends of a polyester with a minor portion of another thermoplastic material, polyolefine, styrenic block polymers, polyester and polyurethane.

The at least one crosslinking agent is advantageously selected from the group comprising: aryl azide, diazirines, benzophenone, azobisisobutyronitrile, benzoyl peroxide and camphorquinone.

In a preferred embodiment of the invention benzophenone derivatives are used as crosslinking agent. Benzophenones are advantageously used as these compounds can be activated multiple times and can additionally act as adhesion promoter at the interface between adhesive layers and radically molding coatings.

According to a preferred embodiment of the invention the balloon is made of a polyamide based thermoplastic elastomer, in particular PEBAX, with a shore hardness below 70 and/or VESTAMID ME with a shore hardness below 70, which are building a compound with a crosslinking agent activated by UV light and suitable for a thermoplastic process.

According to another aspect the invention is related to a balloon made of a mixture of at least one polymer and at least one crosslinking agent, whereby polymer chains are connected by the crosslinking agent, which is activated by irradiation with UV light. The at least one crosslinking agent is consequently sensitive to irradiation by UV light.

The inventive balloon is preferably used as a part of a medical device, in particular a component of a catheter. More preferred the inventive balloon is used as component of a balloon catheter suitable for introducing into a body vessel, in particular suitable to performing a balloon angioplasty, implanting an endoprosthesis, in particular a stent, and/or implanting a valve prosthesis.

It will be apparent to those skilled in the art that numerous modifications and variations of the described examples and embodiments are possible in light of the above teaching. The disclosed examples and embodiments are presented for purposes of illustration only. Other alternate embodiments may include some or all of the features disclosed herein. Therefore, it is the intent to cover all such modifications and alternate embodiments as may come within the true scope of this invention.

It will be apparent to those skilled in the art that numerous modifications and variations of the described examples and embodiments are possible in light of the above teaching. The disclosed examples and embodiments are presented for purposes of illustration only. Other alternate embodiments may include some or all of the features disclosed herein. Therefore, it is the intent to cover all such modifications and alternate embodiments as may come within the true scope of this invention.

## Claims

**1.** Method for manufacturing a balloon catheter, in particular usable for introducing into a body vessel, whereby the balloon is made of a mixture of at least one polymer and the least one crosslinking agent, **characterized in that** the claimed method comprises a step, whereby the balloon is irradiated by UV light.

**2.** Method according to claim 1, **characterized in that** said balloon is formed by blow moulding a balloon preform, fastened to a shaft of said catheter and irradiated by UV light.

**3.** Method according to claim 2, **characterized in that** said balloon preform is produced by an extrusion process, in particular by extruding a granulate material of a compound comprising at least one polymer and at least one crosslinking agent.

**3.** Method according to claim 2 or 3, **characterized in that** the balloon is adhesively bonded to a shaft of said catheter, whereby the adhesive bonding is achieved by UV light irradiation of a suitable compound.

**4.** Method according to any of the preceding claims, **characterized in that** at least one polymer is selected from the group comprising: PET (Polyethylene terephthalate), PEBA (polyether block amide, e.g Vestamid E or PEBAX,), in particular a polyamide, in particular polyamide 12, nylon, polyester copolymers, blends of polyesters or blends of a polyester with a minor portion of another thermoplastic material, polyolefine, styrenic block polymers, polyester and polyurethane

**5.** Method according to any of the preceding claims, **characterized in that** at least one crosslinking agent is selected from the group comprising: aryl azide, diazirines, benzophenone, azobisisobutyronitrile, benzoyl peroxide and camphorquinone

**6.** Balloon made of a mixture of at least one polymer and at least one crosslinking agent, **characterized in that** polymer chains are connected by the crosslinking agent, which is activated by irradiation with UV light.

**7.** Balloon according to claim 6, **characterized in that**, at least one crosslinking agent is sensitive to irradiation by UV light.

**8.** Use of a balloon according to claim 6 or 7, **characterized in that** said balloon is a part of a medical device, in particular a component of a catheter.

**9.** Use of a balloon according to claim 8 as component of a balloon catheter suitable for introducing into a body vessel, in particular suitable to performing a balloon angioplasty, implanting an endoprosthesis, in particular a stent, and/or implanting a valve prosthesis.
